# EUROPÄISCHE PATENTSCHRIFT

(11) **EP 2 776 067 B1**
(45) Veröffentlichungstag und Bekanntmachung des Hinweises auf die Patenterteilung: **21.12.2016**
(21) Anmeldenummer: 12781328.5
(22) Anmeldetag: 07.11.2012
(51) Int. Cl.: A61K 41/00, A61K 47/42, A61P 35/00, A61K 31/409, A61K 49/00

(54) **ZUSAMMENSETZUNG FÜR DIE PHOTODYNAMISCHE DIAGNOSTIK UND THERAPIE VON TUMOREN**
COMPOSITION FOR THE PHOTODYNAMIC DIAGNOSIS AND TREATMENT OF TUMOURS
COMPOSITION POUR LE DIAGNOSTIC ET LE TRAITEMENT PHOTODYNAMIQUE DES TUMEURS

(30) Priorität: 09.11.2011 EP 11188495
(43) Veröffentlichungstag der Anmeldung: 17.09.2014
(73) Patentinhaber: APOCARE Pharma GmbH, 33647 Bielefeld (DE)
(72) Erfinder: HÜTTENBERGER, Dirk, 67659 Kaiserslautern (DE); HAUPT, Manfred, 33615 Bielefeld (DE)
(74) Vertreter: Weickmann & Weickmann PartmbB
(86) Internationale Anmeldenummer: PCT/EP2012/072039
(87) Internationale Veröffentlichungsnummer: WO 2013/068405

(56) Entgegenhaltungen:
- WO-A2-2011/071968
- JEONG HAYOUNG ET AL: "Photosensitizer-conjugated human serum albumin nanoparticles for effective photodynamic therapy.", THERANOSTICS 2011 LNKD- PUBMED:21562630, Bd. 1, 6. April 2011 (2011-04-06), Seiten 230-239, XP002668309, ISSN: 1838-7640
- MOJZISOVA ET AL: "The pH-dependent distribution of the photosensitizer chlorin e6 among plasma proteins and membranes: A physico-chemical approach", BIOCHIMICA ET BIOPHYSICA ACTA. BIOMEMBRANES, AMSTERDAM, NL, Bd. 1768, Nr. 2, 25. Januar 2007 (2007-01-25), Seiten 366-374, XP005859036, ISSN: 0005-2736, DOI: 10.1016/J.BBAMEM.2006.10.009
- OL'SHEVSKAYA V A ET AL: "Novel boronated chlorin e6-based photosensitizers: Synthesis, binding to albumin and antitumour efficacy", BIOORGANIC & MEDICINAL CHEMISTRY, PERGAMON, GB, Bd. 17, Nr. 3, 1. Februar 2009 (2009-02-01), Seiten 1297-1306, XP025925757, ISSN: 0968-0896, DOI: 10.1016/J.BMC.2008.12.016 [gefunden am 2009-01-31]
- KHADEM ET AL: "Photodynamic tissue adhesion with chlorin(e6) protein conjugates.", INVESTIGATIVE OPHTHALMOLOGY & VISUAL SCIENCE, Bd. 40, Nr. 13, 1. Dezember 1999 (1999-12-01), Seiten 3132-3137, XP055017821, ISSN: 0146-0404

## Beschreibung

Die vorliegende Erfindung betrifft eine Zusammensetzung, welche insbesondere in der photodynamischen Diagnostik oder/und Therapie von Tumoren eingesetzt werden kann. Weiterhin betrifft die Erfindung die Verwendung von Albuminen zur Stabilisierung von Chlorin e6 oder pharmazeutisch annehmbaren Derivaten hiervon.

Die photodynamische Diagnostik (PDD) und die photodynamische Therapie (PDT) stellen Verfahren zur Erkennung bzw. Behandlung von Tumoren und anderen Gewebeveränderungen dar, welche auf einer Bestrahlung des betroffenen Gewebes mit Licht geeigneter Wellenlänge basieren. Hierbei wird dem Patienten vor der Bestrahlung ein primär nicht-toxischer Photosensibilisator verabreicht, welcher sich aufgrund einer Reihe von Faktoren (spezifische Transporter, erhöhter Tumorzellstoffwechsel, verstärkte Blutgefäßversorgung, geringer lymphatischer Abfluß) spezifisch in Tumorzellen anreichert.

Nach der maximalen Anreicherung des Photosensibilisators im Tumorgewebe kann dieser durch Bestrahlung mit Licht geeigneter Wellenlänge angeregt werden. Im angeregten Zustand überträgt der Photosensibilisator Energie auf einen geeigneten Reaktionspartner, wie beispielsweise auf im Gewebe vorhandenen molekularen Sauerstoff. Der hierbei entstehende Singulett-Sauerstoff stellt ein starkes Oxidationsmittel dar, welches zelluläre Strukturen von Tumorzellen durch Oxidation so stark schädigt, dass dies zur Apoptose oder Nekrose führt.

Photosensibilisatoren, welche für eine Anwendung im Bereich der photodynamischen Diagnostik bzw. Therapie in Frage kommen, umfassen Hämatoporphyrine, Phthalocyanine und Naphthalocyanine. Aufgrund geringer Toxizität und einer effizienten Anregbarkeit im sichtbaren Spektralbereich haben sich in der Praxis insbesondere Photosensibilisatoren auf Porphyrinbasis bewährt, welche u.a. Chlorin e6 und Derivate hiervon umfassen.

Chlorin e6 besitzt mehrere intensive Absorptionsbanden. Von praktischer Bedeutung sind hierbei insbesondere die sogenannte Soretbande im Spektralbereich von 400 ± 10 nm, welche für die photodynamische Diagnostik Relevanz besitzt, sowie eine Absorptionsbande bei 660 ± 10nm, welche aufgrund einer höheren Eindringtiefe in das Gewebe für die photodynamische Therapie genutzt wird. Gleichzeitig besitzt Chlorin e6 den Vorteil, dass es im Körper vergleichsweise schnell metabolisiert und ausgeschieden wird; 48 Stunden nach der Verabreichung sind nur noch Spuren der verabreichten Wirksubstanz im menschlichen Organismus nachzuweisen.

Allerdings ergibt sich für Chlorin e6 das Problem, dass die Substanz in wässriger Lösung instabil ist. Infolge dessen kann eine gebrauchsfertige wässrige Lösung von Chlorin e6 nicht über längere Zeiträume unter üblichen Bedingungen, d.h. im Kühlschrank oder bei Raumtemperatur, gelagert werden, ohne dass ein signifikanter Abbau der Substanz erfolgt, der zu einem geringerem Wirkstoffgehalt und zur Entstehung unerwünschter Zersetzungsprodukte führt.

Die der vorliegenden Erfindung zugrundeliegende Aufgabe bestand somit darin, eine Zusammensetzung enthaltend Chlorin e6 oder ein pharmazeutisch annehmbares Derivat hiervon bereitzustellen, bei welcher die Nachteile des Standes der Technik zumindest teilweise beseitigt sind. Insbesondere sollte die Zusammensetzung über einen längeren Zeitraum, z.B. über einen Zeitraum von mehreren Wochen bei Raumtemperatur bzw. über einen Zeitraum von mehreren Monaten bei Kühlschranktemperatur, gelagert werden können, ohne dass der Wirkstoff in signifikantem Ausmaß zersetzt wird.

RU2152790C1 offenbart eine Zusammensetzung zur photodynamischen Diagnose und Therapie, die Chlorin e6 sowie Polyvinylpyrrolidon enthält. Polyvinylpyrrolidon dient der Langzeitstabilisierung von Chlorin e6.

Die vorliegende Erfindung löst die oben genannte Aufgabe durch eine Zusammensetzung, welche neben Chlorin e6 oder einem pharmazeutisch annehmbaren Derivat hiervon obligatorisch ein Albumin umfasst.

Der Gegenstand der vorliegenden Erfindung betrifft eine Zusammensetzung, umfassend
(a) Chlorin e6 oder ein pharmazeutisch annehmbares Derivat hiervon, ausgewählt aus pharmazeutisch annehmbaren Salzen, Estern und Amiden, und
b) ein Albumin, wobei das Chlorin e6 oder das pharmazeutisch annehmbare Derivat davon und das Albumin nicht über eine kovalente Bindung miteinander verknüpft sind, und wobei die Zusammensetzung das Albumin in einer Menge von 1,0 Gew.-% bis 20 Gew.-% bezogen auf das Gesamtgewicht der Zusammensetzung umfasst.

Überraschenderweise wurde gefunden, dass mit Hilfe von Albuminen eine Langzeitstabilisierung von Chlorin e6 oder pharmazeutisch annehmbaren Derivaten hiervon möglich ist. Bei dem Albumin kann es sich grundsätzlich um ein beliebiges Albumin natürlichen oder synthetischen Ursprungs handeln, wobei Humanalbumine bevorzugt sind. Stärker bevorzugt handelt es sich bei dem Albumin um humanes Serumalbumin (HSA). Erfindungsgemäß sind Chlorin e6 bzw. dessen pharmazeutisch annehmbares Derivat und das Albumin indessen nicht über eine kovalente Bindung miteinander verknüpft, so dass die erfindungsgemäße Zusammensetzung keinerlei Konjugat aus Chlorin e6 und einem Albumin bzw. keinerlei Konjugat aus einem pharmazeutisch annehmbaren Chlorin e6-Derivat und einem Albumin umfasst.

Der Ausdruck "pharmazeutisch annehmbares Derivat", wie er in vorliegender Anmeldung verwendet wird, bezeichnet einen Abkömmling eines Wirkstoffs, welcher für den Empfänger im wesentlichen unschädlich ist und die biologische Wirksamkeit bzw. die biologischen Eigenschaften des Wirkstoffs besitzt. Beispiele für pharmazeutisch annehmbare Derivate im Sinne der vorliegenden Anmeldung umfassen pharmazeutisch annehmbare Salze, Ester und Amide, welche vorzugsweise kein Bor umfassen und stärker bevorzugt gänzlich frei von Halbmetallen sind. In einer besonders bevorzugten Ausführungsform handelt es sich bei dem pharmazeutisch annehmbaren Derivat um ein pharmazeutisch annehmbares Salz, wie beispielsweise um das Trinatriumsalz von Chlorin e6.

Der Begriff "Langzeitstabilisierung", wie er in vorliegender Anmeldung verwendet wird, bedeutet, dass Chlorin e6 für eine beliebige Zeitspanne, bevorzugt über einen Zeitraum von mindestens 4 Wochen, stärker bevorzugt über einen Zeitraum von mindestens 6 Monaten, und am stärksten bevorzugt über einen Zeitraum von mindestens 12 Monaten in Gegenwart eines Albumins gelagert wird, wobei das Gemisch aus Chlorin e6 und Albumin nach der Lagerung mehr als 65 Gew.-%, bevorzugt mehr als 80 Gew.-%, besonders bevorzugt mehr als 90 Gew.-% der Ausgangsmenge an Chlorin e6 enthält. Die Lagerung erfolgt vorzugsweise bei Atmosphärendruck, bei einer relativen Luftfeuchtigkeit von mindestens 50%, und bei einer Temperatur im Bereich von etwa 2°C bis etwa 25°C (Raumtemperatur), wobei sich eine Lagerung bei 2-8°C als besonders vorteilhaft erwiesen hat.

Die erfindungsgemäße Zusammensetzung kann Chlorin e6 und das Albumin grundsätzlich in jeder dem Fachmann geeignet erscheinenden Menge enthalten. Für die Zwecke der Erfindung ist es indessen bevorzugt, dass die Zusammensetzung Chlorin e6 in einer Menge von etwa 0.02 Gew.-% bis etwa 0.2 Gew.-%, stärker bevorzugt in einer Menge von etwa 0.04 Gew.-% bis etwa 0.16 Gew.-%, und am stärksten bevorzugt in einer Menge von etwa 0.06 Gew.-% bis etwa 0.12 Gew.-%, bezogen auf das Gesamtgewicht der Zusammensetzung, umfasst.

Demgegenüber umfasst die Zusammensetzung das Albumin in einer Menge von etwa 1.0 Gew.-% bis etwa 20 Gew.-%, und bevorzugt in einer Menge von etwa 2.5 Gew.-% bis etwa 15 Gew.-%, bezogen auf das Gesamtgewicht der Zusammensetzung.

Die erfindungsgemäße Zusammensetzung kann grundsätzlich beliebig formuliert sein, sofern die Formulierung dem Fachmann für den beabsichtigten Verwendungszweck geeignet erscheint. Beispiele für Formulierungen im Sinne der vorliegenden Anmeldung umfassen u.a. Aerosole, Lösungen, Schäume, Emulsionen, Suspensionen, Gele, Cremes, Salben, Lotionen, Tabletten und Zäpfchen, sind jedoch nicht auf diese beschränkt. Bevorzugt ist die Zusammensetzung als Lösung formuliert, wobei wässrige Lösungen als besonders bevorzugt gelten.

Die erfindungsgemäße Zusammensetzung kann, sofern sie in flüssiger Form vorliegt, grundsätzlich jeden vom Fachmann für sinnvoll erachteten pH-Wert aufweisen. Da die Stabilität von Chlorin e6 mit sinkendem pH-Wert abnimmt, ist es erfindungsgemäß allerdings bevorzugt, dass die Zusammensetzung einen pH-Wert von ≥ 6.0 aufweist. Stärker bevorzugt weist die Zusammensetzung einen pH-Wert im Bereich von 6.0 bis 10.0, noch stärker bevorzugt einen pH-Wert im Bereich von 7.0 bis 9.0 auf. Als besonders bevorzugt gilt ein pH-Wert im Bereich von 8.0 bis 9.0.

Neben Chlorin e6 und einem Albumin kann die Zusammensetzung, sofern gewünscht, weitere Komponenten enthalten, wie beispielsweise einen pharmazeutisch annehmbaren Träger oder/und Additive. In einer bevorzugten Ausführungsform der Erfindung umfasst die Zusammensetzung indessen keinerlei Pyrrolidon-Derivate, wie beispielsweise kein Polyvinylpyrrolidon. Besonders bevorzugt ist die erfindungsgemäße Zusammensetzung frei von Stabilisatoren, wobei der Begriff "Stabilisator", wie er in vorliegender Anmeldung verwendet wird, jegliches chemische oder physikalische Mittel umfasst, das vom Fachmann üblicherweise zur Stabilisierung chemischer Systeme eingesetzt wird.

Der Ausdruck "pharmazeutisch annehmbarer Träger", wie er in vorliegender Anmeldung verwendet wird, bezeichnet jegliche organische oder anorganische, natürliche oder synthetische Substanz, welcher zur Vereinfachung der Applikation eines Wirkstoffs mit diesem kombiniert werden kann und für eine Verabreichung an Säuger, einschließlich Menschen, geeignet ist. Beispiele für pharmazeutisch annehmbare Träger umfassen, sind jedoch nicht beschränkt auf, organische oder anorganische Lösungsmittel, Stärke, Laktose, Mannitol, Methylcellulose, Talk, Gelatine, Agar-Agar, Calciumphosphat, Magnesiumstearat, tierische und pflanzliche Fette, höhermolekulare Fettsäuren, oder höhermolekulare Polymere.

Additive im Sinne der vorliegenden Anmeldung umfassen beispielsweise pH-Puffer, Verdünnungsmittel, Verarbeitungshilfsmittel wie etwa Emulgatoren, Konservierungsmittel, Stabilisatoren, Antioxidationsmittel, Lichtschutzmittel, und Farbstoffe, sind jedoch nicht auf diese beschränkt. In einer bevorzugten Ausführungsform der Erfindung umfasst die Zusammensetzung neben Chlorin e6 und einem Albumin weiterhin einen pH-Puffer, welcher vom Fachmann entsprechend den Anforderungen an die zu applizierende Zusammensetzung ausgewählt werden kann. Beispielhafte pH-Puffer umfassen, sind jedoch nicht beschränkt auf, Bicarbonatpuffer, Phosphatpuffer, und dergleichen.

In einem weiteren Aspekt betrifft die Erfindung die erfindungsgemäßen Zusammensetzung zur Verwendung in einem medizinischen Verfahren.

Bevorzugt gelangt die erfindungsgemäße Zusammensetzung hierbei in der photodynamischen Diagnostik oder/und Therapie von dysplastischen Veränderungen, und besonders bevorzugt in der photodynamischen Diagnostik oder/und Therapie von Tumoren, zum Einsatz. Alternativ kann die erfindungsgemäße Zusammensetzung auch zur Bekämpfung von Bakterien eingesetzt werden, wobei der Begriff "Bekämpfung" sowohl eine bloße Inaktivierung als auch eine vollständige Abtötung umfasst. Die Bekämpfung der Bakterien kann in vivo oder ex vivo erfolgen.

Sofern die Zusammensetzung in der photodynamischen Diagnostik oder/und Therapie von Tumoren verwendet wird, so handelt es sich bei dem Tumor bevorzugt um einen Tumor der Blase, der Prostata, der Lunge, der Schleimhäute, des Hals-Kopf-Bereichs, oder der Haut. Besonders bevorzugt können mittels der Zusammensetzung Tumore der Haut, welche speziell Basaliome und Melanome umfassen, diagnostiziert oder/und behandelt werden.

In noch einem weiteren Aspekt betrifft die Erfindung die Verwendung von Albuminen zur Stabilisierung von Chlorin e6 oder pharmazeutisch annehmbaren Derivaten hiervon, ausgewählt aus pharmazeutisch annehmbaren Salzen, Estern und Amiden, wobei das Albumin und das Chlorin e6 oder das pharmazeutisch annehmbare Derivat davon nicht über eine kovalente Bindung miteinander verknüpft sind und wobei das Albumin in einer Menge von 1,0 Gew.% bis 20 Gew.% bezogen auf das Gesamtgewicht der Zusammensetzung eingesetzt wird. Was bevorzugte Ausgestaltungen des Albumins betrifft, so wird auf die Ausführungen im Rahmen der Beschreibung der erfindungsgemäßen Zusammensetzung verwiesen.

Die Erfindung soll durch die nachfolgenden Figuren und Beispiele näher erläutert werden:

### Beschreibung der Figuren

**Figur 1****:** Stabilität verschiedener Zusammensetzungen gemäß der vorliegenden Erfindung bei einem pH-Wert von 7.0, 7.5 bzw. 8.0 nach Lagerung für 4 Tage bei 30°C.
**Figur 2****:** Stabilität verschiedener Zusammensetzungen gemäß der vorliegenden Erfindung bei einem pH-Wert von 7.0, 7.5 bzw. 8.0 nach Lagerung für 4 Wochen bei 30°C.
**Figur 3****:** Stabilität verschiedener Zusammensetzungen gemäß der vorliegenden Erfindung bei einem pH-Wert von 7.0, 7.5 bzw. 8.0 nach Lagerung für 10 Wochen bei 30°C.
**Figur 4****:** Stabilität verschiedener Zusammensetzungen gemäß der vorliegenden Erfindung bei einem pH-Wert von 8.0 nach Lagerung für 6 Monate bei 5°C.
**Figur 5****:** Stabilität verschiedener Zusammensetzungen gemäß der vorliegenden Erfindung bei einem pH-Wert von 8.0 nach Lagerung für 9 Monate bei 5°C.
**Figur 6****:** Stabilität verschiedener Zusammensetzungen gemäß der vorliegenden Erfindung bei einem pH-Wert von 8.0 nach Lagerung für 12 Monate bei 5°C.

### Beispiel

Zur Bestimmung der Stabilität einer Zusammensetzung, umfassend Chlorin e6 und ein Albumin, wurden zunächst drei Pufferlösungen mit pH-Werten von 7.0, 7.5 bzw. 8.0, welche jeweils Kaliumdihydrogenphosphat (Firma Merck) und Dinatriumhydrogenphosphat (Firma Merck) als Puffersalze enthielten, hergestellt und sterilisiert.

Ausgehend von einer 20 Gew.-% Stammlösung von humanem Serumalbumin (Firma Biotest AG) wurden mittels obiger Pufferlösungen anschließend jeweils verdünnte Lösungen mit 10 Gew.-%, 5 Gew.-% bzw. 0 Gew.-% an humanem Serumalbumin hergestellt. Zum Puffern der 20 Gew.-% Stammlösung von humanem Serumalbumin wurden die Puffersalze als Feststoffe direkt zu 20 ml Aliquoten der Stammlösung hinzugefügt.

In der Folge wurden 2 ml der verschiedenen gepufferten Lösungen jeweils in Braunglasflaschen mit Bördelverschluss eingebracht und mit 40 µl einer 75 mM Lösung von Chlorin e6-Natriumsalz (Fa. ORPEGEN Peptide Chemicals GmbH) in Wasser versetzt. Auf diese Weise wurden wässrige Lösungen von Chlorin e6-Natriumsalz mit einer Konzentration von 0 Gew.-%, 1.25 Gew.-%, 2.5 Gew.-%, 5 Gew.-%, 10 Gew.-% bzw. 20 Gew.-% an humanem Serumalbumin bereitgestellt, welche pH-Werte von 7.0, 7.5 bzw. 8.0 aufwiesen. Zwecks Reproduzierbarkeit der Ergebnisse wurden die einzelnen Lösungen jeweils mehrfach angesetzt.

Die Lösungen wurden lichtdicht umverpackt und bei 30°C in einem Trockenschrank bzw. bei 5°C in einem Kühlschrank inkubiert. Nach 4 Tagen, 4 Wochen und 10 Wochen Lagerung im Trockenschrank bzw. nach 6 Monaten Lagerung im Kühlschrank wurde jeweils eine Probe der jeweiligen Lösung genommen und mittels analytischer HPLC (Säule: C18, 250 mm x 4.5 mm (Dr. Maisch GmbH); Laufmittel: 80 mM Triethylammoniumphosphat-Puffer pH 2.25/Acetonitril) vermessen. Das jeweilige Ausmaß der Zersetzung von Chlorin e6 wurde durch einen Vergleich der Flächen unter dem zu Chlorin e6 gehörigen Peak im HPLC-Diagramm vor und nach der Lagerung berechnet.

Bei der Auswertung von Proben, welche nach 4 Tagen Lagerung genommen wurden, zeigte sich in einer auf pH 8 eingestellten reinen Pufferlösung, d.h. in Abwesenheit von humanem Serumalbumin, ein signifikanter Abbau von Chlorin e6 (59.1% Fläche). Da kommerziell erhältliche Lösungen von humanem Serumalbumin obligatorisch Na-caprylat und N-Acetyl-DL-Tryptophan als Additive enthalten, wurde ferner ein Versuch durchgeführt, in welchem der Einfluss der beiden Substanzen auf die Stabilität einer wässrigen Lösung von Chlorin e6 untersucht wurde. Dabei zeigte sich, dass durch Zugabe von Na-caprylat bzw. N-Acetyl-DL-Tryptophan der Wirkstoff gegenüber der reinen Pufferlösung eine leichte Stabilisierung erfährt (68.8% Fläche).

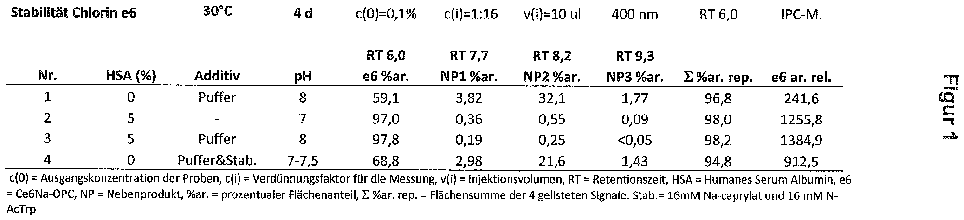

Der geringste Abbau von Chlorin e6 wurde in einer auf pH 8 eingestellten Pufferlösung beobachtet, welche 5 Gew.-% humanes Serumalbumin enthielt (97.8% Fläche). Im Falle einer Lagerung des Wirkstoffs in einer auf pH 7 eingestellten, nicht-gepufferten Lösung wurde in Gegenwart von 5% humanem Serumalbumin eine gegenüber Na-caprylat und N-Acetyl-DL-Tryptophan deutlich bessere Stabilisierung von Chlorin e6 beobachtet (97.0% Fläche). Die Ergebnisse sind in Figur 1 dargestellt.

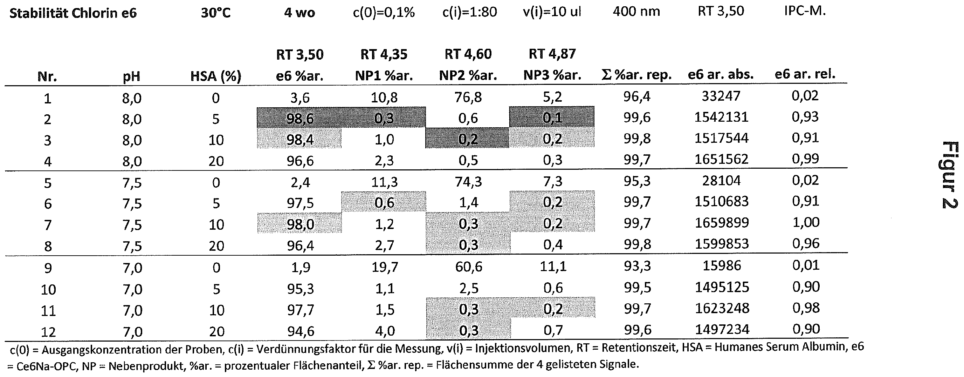

Die Vermessung von Proben nach 4 Wochen Lagerung im Trockenschrank zeigte im Falle reiner Pufferlösungen, d.h. ohne Zusatz von humanem Serumalbumin, einen nahezu vollständigen Abbau von Chlorin e6 (pH 7.0: 1.9% Fläche; pH 7.5: 2.4% Fläche; pH 8.0: 3.6% Fläche). Durch Zugabe von 5 Gew.-% an humanem Serumalbumin konnte die Zersetzung von Chlorin e6 bei allen drei pH-Werten signifikant reduziert werden, wie durch einen Restwirkstoffgehalt von > 95% belegt ist (pH 7.0: 95.3% Fläche; pH 7.5: 97.5% Fläche; pH 8.0: 98.6% Fläche). Bei Erhöhung der Menge an humanem Serumalbumin auf 10 Gew.-% wurde bei pH 7 und pH 7.5 ein etwas höherer Restwirkstoffgehalt beobachtet; bei Inkubation mit 20 Gew.-% humanem Serumalbumin sank der Restgehalt an Chlorin e6 bei allen drei pH-Werten leicht (pH 7.0: 94.6% Fläche; pH 7.5: 96.4% Fläche; pH 8.0: 96.6% Fläche). Die Ergebnisse sind in Figur 2 dargestellt.

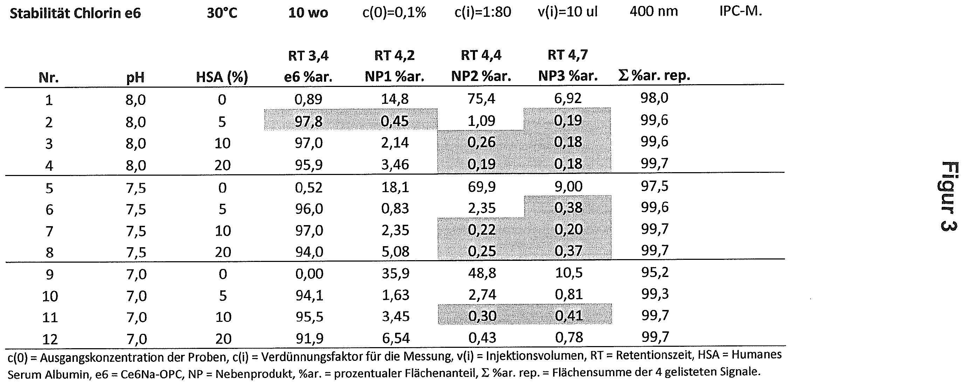

Die Vermessung von Proben nach 10 Wochen Lagerung bei 30°C zeigte im Falle reiner Pufferlösungen einen quasi vollständigen Abbau von Chlorin e6 (pH 7.0: 0% Fläche; pH 7.5: 0.52% Fläche; pH 8.0: 0.89% Fläche). Durch Zugabe von 5 Gew.-% an humanem Serumalbumin konnte die Zersetzung von Chlorin e6 bei allen drei pH-Werten wiederum signifikant reduziert werden, wie durch einen Restwirkstoffgehalt von > 94% belegt ist (pH 7.0: 94.1% Fläche; pH 7.5: 96.0% Fläche; pH 8.0: 97.8% Fläche). Bei Erhöhung der Menge an humanem Serumalbumin auf 10 Gew.-% wurde bei pH 7 und pH 7.5 erneut ein etwas höherer Restwirkstoffgehalt beobachtet; bei Inkubation mit 20 Gew.-% humanem Serumalbumin sank der Restgehalt an Chlorin e6 bei allen drei pH-Werten (pH 7.0: 91.9% Fläche; pH 7.5: 94.0% Fläche; pH 8.0: 95.9% Fläche). Die Ergebnisse, welche die Ergebnisse nach 4 Wochen auf etwas niedrigerem Chlorin e6-Niveau widerspiegeln, sind in Figur 3 dargestellt.

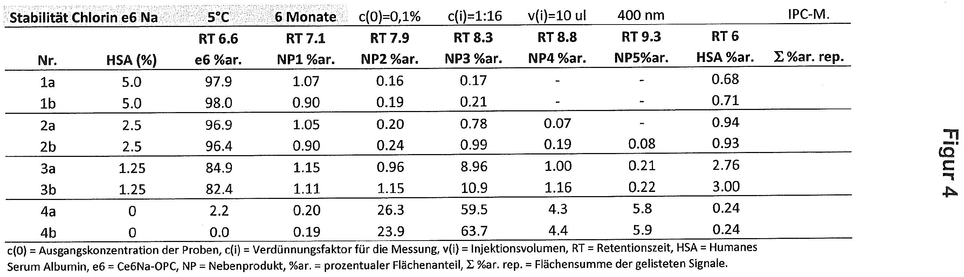

Die Vermessung von Proben nach 6 Monaten Lagerung bei 5°C und pH 8.0 zeigte im Falle reiner Pufferlösungen einen nahezu vollständigen Abbau von Chlorin e6 (1.1% Fläche im Mittel). Durch Zugabe von 5 Gew.-% an humanem Serumalbumin konnte die Zersetzung von Chlorin e6 signifikant reduziert werden, wie durch einen Restwirkstoffgehalt von > 97% belegt ist (97.9% Fläche im Mittel). Bei Erniedrigung der Menge an humanem Serumalbumin auf 2.5 Gew.-% wurde ein etwas niedrigerer Restwirkstoffgehalt von etwa 96.6% (Mittelwert) beobachtet; bei Inkubation mit 1.25 Gew.-% humanem Serumalbumin sank der Restgehalt an Chlorin e6 auf etwa 83.6% (Mittelwert). Die Ergebnisse sind in Figur 4 dargestellt.

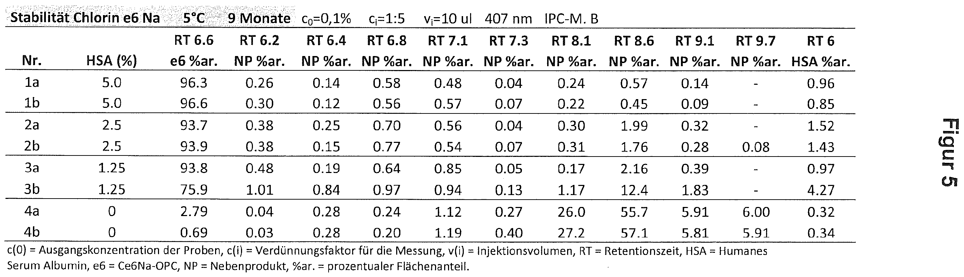

Die Vermessung von Proben nach 9 Monaten Lagerung bei 5°C und pH 8.0 zeigte im Falle reiner Pufferlösungen ebenfalls einen nahezu vollständigen Abbau von Chlorin e6 (1.74% Fläche im Mittel). Durch Zugabe von 5 Gew.-% an humanem Serumalbumin konnte die Zersetzung von Chlorin e6 signifikant reduziert werden, wie durch einen Restwirkstoffgehalt von > 96% belegt ist (96.4% Fläche im Mittel). Bei Erniedrigung der Menge an humanem Serumalbumin auf 2.5 Gew.-% wurde ein etwas niedrigerer Restwirkstoffgehalt von etwa 93.8% (Mittelwert) beobachtet; bei Inkubation mit 1.25 Gew.-% humanem Serumalbumin sank der Restgehalt an Chlorin e6 auf etwa 84.9% (Mittelwert). Die Ergebnisse sind in Figur 5 dargestellt.

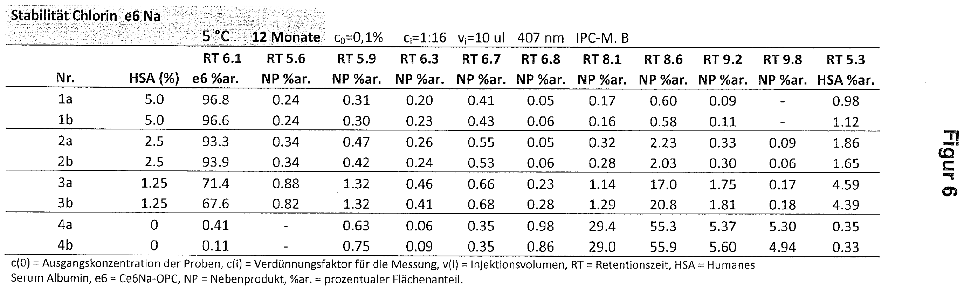

Die Vermessung von Proben nach 12 Monaten Lagerung bei 5°C und pH 8.0 zeigte im Falle reiner Pufferlösungen einen praktisch vollständigen Abbau von Chlorin e6 (0.26% Fläche im Mittel). Durch Zugabe von 5 Gew.-% an humanem Serumalbumin konnte die Zersetzung von Chlorin e6 signifikant reduziert werden, wie durch einen Restwirkstoffgehalt von > 96% belegt ist (96.7% Fläche im Mittel). Bei Erniedrigung der Menge an humanem Serumalbumin auf 2.5 Gew.-% wurde ein etwas niedrigerer Restwirkstoffgehalt von etwa 93.6% (Mittelwert) beobachtet; bei Inkubation mit 1.25 Gew.-% humanem Serumalbumin sank der Restgehalt an Chlorin e6 auf etwa 69.5% (Mittelwert). Die Ergebnisse sind in Figur 6 dargestellt.

Zusammenfassend bedeutet dies, dass bereits geringe Mengen an humanem Serumalbumin eine Chlorin e6-enthaltende wässrige Lösung drastisch stabilisieren. Sofern die Lagerung der Zusammensetzung bei einer Temperatur von 2-8°C anstelle von 30°C erfolgt, lässt sich aus den in den Figuren 1-6 gezeigten experimentellen Daten eine Haltbarkeit von mindestens 12 Monaten für eine wässrige Lösung von Chlorin e6 extrapolieren, womit die Zusammensetzung eine für medizinische Anwendungen geeignete Langzeitstabilität besitzt.

## Patentansprüche

1. Zusammensetzung, umfassend
(a) Chlorin e6 oder ein pharmazeutisch annehmbares Derivat hiervon, ausgewählt aus pharmazeutisch annehmbaren Salzen, Estern und Amiden, und
b) ein Albumin, wobei das Chlorin e6 oder das pharmazeutisch annehmbare Derivat davon und das Albumin nicht über eine kovalente Bindung miteinander verknüpft sind, und wobei die Zusammensetzung das Albumin in einer Menge von 1,0 Gew.-% bis 20 Gew.-% bezogen auf das Gesamtgewicht der Zusammensetzung umfasst.

2. Zusammensetzung nach Anspruch 1,
**dadurch gekennzeichnet,**
**dass** es sich bei dem Albumin um humanes Serumalbumin handelt.

3. Zusammensetzung nach Anspruch 1 oder 2,
**dadurch gekennzeichnet,**
**dass** sie Chlorin e6 in einer Menge von etwa 0.02 Gew.-% bis etwa 0.2 Gew.-%, bevorzugt in einer Menge von etwa 0.06 Gew.-% bis etwa 0.12 Gew.-%, bezogen auf das Gesamtgewicht der Zusammensetzung, umfasst.

4. Zusammensetzung nach einem der Ansprüche 1 bis 3,
**dadurch gekennzeichnet,**
**dass** sie das Albumin in einer Menge von etwa 2.5 Gew.-% bis etwa 15 Gew.-%, bezogen auf das Gesamtgewicht der Zusammensetzung, umfasst.

5. Zusammensetzung nach einem der Ansprüche 1 bis 4,
**dadurch gekennzeichnet,**
**dass** sie als wässrige Lösung formuliert ist.

6. Zusammensetzung nach einem der Ansprüche 1 bis 5,
**dadurch gekennzeichnet,**
**dass** sie einen pH-Wert im Bereich von 6.0 bis 10.0, bevorzugt im Bereich von 8.0 bis 9.0, aufweist.

7. Zusammensetzung nach einem der Ansprüche 1 bis 6,
**dadurch gekennzeichnet,**
**dass** sie weiterhin einen pH-Puffer umfasst.

8. Zusammensetzung nach einem der Ansprüche 1 bis 7,
**dadurch gekennzeichnet,**
**dass** sie kein Pyrrolidon-Derivat umfasst.

9. Zusammensetzung nach einem der Ansprüche 1 bis 8 zur Verwendung in einem medizinischen Verfahren.

10. Zusammensetzung nach einem der Ansprüche 1 bis 8 zur Verwendung in der photodynamischen Diagnostik oder/und Therapie von Tumoren.

11. Zusammensetzung zur Verwendung nach Anspruch 10,
**dadurch gekennzeichnet,**
**dass** es sich bei dem Tumor um einen Tumor der Blase, der Prostata, der Lunge, der Schleimhäute, des Hals-Kopf-Bereichs oder der Haut, insbesondere der Haut, handelt.

12. Zusammensetzung nach einem der Ansprüche 1 bis 8 zur Verwendung in einem Verfahren zur Bekämpfung von Bakterien.

13. Verwendung eines Albumins zur Stabilisierung von Chlorin e6 oder einem pharmazeutisch annehmbaren Derivat hiervon ausgewählt aus pharmazeutisch annehmbaren Salzen, Estern und Amiden, wobei das Albumin und das Chlorin e6 oder das pharmazeutisch annehmbare Derivat davon nicht über eine kovalente Bindung miteinander verknüpft sind und wobei das Albumin in einer Menge von 1,0 Gew.-% bis 20 Gew.-% bezogen auf das Gesamtgewicht der Zusammensetzung eingesetzt wird.

14. Verwendung nach Anspruch 13,
**dadurch gekennzeichnet,**
**dass** es sich bei dem Albumin um humanes Serumalbumin handelt.

## Claims

1. Composition comprising:
(a) chlorin e6 or a pharmaceutically acceptable derivative thereof, selected from pharmaceutically acceptable salts, esters and amides, and
(b) an albumin, wherein the chlorin e6 or the pharmaceutically acceptable derivative thereof and the albumin are not connected to one another by means of a covalent bond, and
wherein the composition comprises the albumin in a quantity of from 1 wt.% to 20 wt.% based on the total weight of the composition.

2. Composition according to claim 1, **characterised in that** the albumin is human serum albumin.

3. Composition according to either claim 1 or claim 2, **characterised in that** it comprises chlorin e6 in a quantity of from approx. 0.02 wt.% to approx. 0.2 wt.%, preferably in a quantity of from approx. 0.06 wt.% to approx. 0.12 wt.% based on the total weight of the composition.

4. Composition according to any of claims 1 to 3, **characterised in that** it comprises the albumin in a quantity of from approx. 2.5 wt.% to approx. 15 wt.% based on the total weight of the composition.

5. Composition according to any of claims 1 to 4, **characterised in that** it is formulated as an aqueous solution.

6. Composition according to any of claims 1 to 5, **characterised in that** it has a pH value in the range of from 6.0 to 10.0, preferably in the range of from 8.0 to 9.0.

7. Composition according to any of claims 1 to 6, **characterised in that** it also has a pH buffer.

8. Composition according to any of claims 1 to 7, **characterised in that** it does not comprise any pyrrolidone derivatives.

9. Composition according to any of claims 1 to 8 for use in a medical procedure.

10. Composition according to any of claims 1 to 8 for use in the photodynamic diagnosis and/or treatment of tumours.

11. Composition for use according to claim 10, **characterised in that** the tumour is a tumour of the bladder, prostate, lungs, mucous membranes, throat-head region or skin, in particular of the skin.

12. Composition according to any of claims 1 to 8 for use in a method for fighting bacteria.

13. Use of an albumin for stabilising chlorin e6 or a pharmaceutically acceptable derivative thereof selected from pharmaceutically acceptable salts, esters and amides, wherein the albumin and the chlorin e6 or the pharmaceutically acceptable derivative thereof are not connected to one another by means of a covalent bond and wherein the albumin is used in a quantity of from 1 wt.% to 20 wt.% based on the total weight of the composition.

14. Use according to claim 13, **characterised in that** the albumin is human serum albumin.

## Revendications

1. Composition, comprenant
a) de la chlorine e6 ou un dérivé pharmaceutiquement acceptable de celle-ci, choisi parmi les sels, les esters et les amides pharmaceutiquement acceptables, et
b) une albumine, dans laquelle la chlorine e6 ou le dérivé pharmaceutiquement acceptable de celle-ci et l'albumine ne sont pas liés l'un à l'autre via une liaison covalente et dans laquelle la composition comprend l'albumine en une quantité de 1,0% en poids à 20% en poids par rapport au poids total de la composition.

2. Composition selon la revendication 1, **caractérisée en ce qu'**il s'agit, pour l'albumine, d'une albumine sérique humaine.

3. Composition selon la revendication 1 ou 2, **caractérisée en ce qu'**elle comprend la chlorine e6 en une quantité d'environ 0,02% en poids à environ 0,2% en poids, de préférence en une quantité d'environ 0,06% en poids à environ 0,12% en poids, par rapport au poids total de la composition.

4. Composition selon l'une quelconque des revendications 1 à 3, **caractérisée en ce qu'**elle comprend l'albumine en une quantité d'environ 2,5% en poids à environ 15% en poids, par rapport au poids total de la composition.

5. Composition selon l'une quelconque des revendications 1 à 4, **caractérisée en ce qu'**elle est formulée sous forme de solution aqueuse.

6. Composition selon l'une quelconque des revendications 1 à 5, **caractérisée en ce qu'**elle présente un pH dans la plage de 6,0 à 10,0, de préférence dans la plage de 8,0 à 9,0.

7. Composition selon l'une quelconque des revendications 1 à 6, **caractérisée en ce qu'**elle comprend en outre un tampon de pH.

8. Composition selon l'une quelconque des revendications 1 à 7, **caractérisée en ce qu'**elle ne comprend pas de dérivé de pyrrolidone.

9. Composition selon l'une quelconque des revendications 1 à 8 pour une utilisation dans un procédé médical.

10. Composition selon l'une quelconque des revendications 1 à 8 pour une utilisation dans le diagnostic photodynamique et/ou la thérapie de tumeurs.

11. Composition pour une utilisation selon la revendication 10, **caractérisée en ce qu'**il s'agit, pour la tumeur, d'une tumeur de la vessie, de la prostate, des poumons, des muqueuses, de la zone du cou et de la tête ou de la peau, en particulier de la peau.

12. Composition selon l'une quelconque des revendications 1 à 8 pour une utilisation dans un procédé pour lutter contre les bactéries.

13. Utilisation d'une albumine pour la stabilisation de chlorine e6 ou d'un dérivé pharmaceutiquement acceptable de celle-ci, choisi parmi les sels, les esters et les amides pharmaceutiquement acceptables, l'albumine et la chlorine e6 ou le dérivé pharmaceutiquement acceptable de celle-ci n'étant pas liés l'un à l'autre via une liaison covalente et l'albumine étant utilisée en une quantité de 1,0% en poids à 20% en poids par rapport au poids total de la composition.

14. Utilisation selon la revendication 13, **caractérisée en ce qu'**il s'agit, pour l'albumine, d'une albumine sérique humaine.
